# EUROPÄISCHE PATENTSCHRIFT

(11) **EP 2 618 920 B1**
(45) Veröffentlichungstag und Bekanntmachung des Hinweises auf die Patenterteilung: **18.06.2014**
(21) Anmeldenummer: 11761273.9
(22) Anmeldetag: 10.08.2011
(51) Int. Cl.: B01F 13/00, B01F 15/02, A61F 2/46, B05C 17/005, A61B 17/88, B05B 1/16, B65D 83/00, A61B 17/00

(54) **KARTUSCHENSYSTEM MIT VORRICHTUNG ZUM SYNCHRONISIEREN ZWEIER FLUIDSTRÖME UND MISCHVERFAHREN**
CARTRIDGE SYSTEM WITH DEVICE FOR SYNCHRONIZING TWO STREAMS OF FLUID AND RELATED METHODS
SYSTÈME DE CARTOUCHE ÉQUIPÉ D'UN DISPOSITIF POUR LA SYNCHRONISATION DE DEUX ÉCOULEMENTS DE FLUIDES ET PROCÉDÉS CORRESPONDANTS

(30) Priorität: 22.09.2010 DE 102010046056
(43) Veröffentlichungstag der Anmeldung: 31.07.2013
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE)
(74) Vertreter: Schultheiss & Sterzel Patentanwälte
(86) Internationale Anmeldenummer: PCT/EP2011/003994
(87) Internationale Veröffentlichungsnummer: WO 2012/038004

(56) Entgegenhaltungen:
- DE-U1-202006 015 457
- GB-A- 2 064 664
- US-A1- 2004 257 909

## Beschreibung

Die Erfindung betrifft ein Kartuschensystem zum Mischen und Applizieren zweier Fluide, insbesondere eines medizinischen Zements, umfassend zwei Kartuschen und eine Austragsöffnung, wobei die Kartuschen durch Kartuschenwände und durch einen Kartuschenkopf begrenzt sind, wobei im Kartuschenkopf wenigstens jeweils eine Kartuschenöffnung zum Austreiben der Kartuscheninhalte aus beiden Kartuschen vorgesehen ist.

Die Erfindung betrifft auch eine Vorrichtung für solch ein erfindungsgemäßes Kartuschensystem, die drehbar oder verschiebbar auf einem Kartuschenkopf eines Kartuschensystems anordbar ist, ein Verfahren zur Synchronisierung von Fluidströmen mit einem erfindungsgemäßen Kartuschensystem oder mit einer erfindungsgemäßen Vorrichtung für ein erfindungsgemäßes Kartuschensystem sowie ein Verfahren zum Mischen eines Mischguts mit einem erfindungsgemäßen Kartuschensystem.

Gegenstand der Erfindung ist also auch eine Vorrichtung zum Synchronisieren von Fluidströmen. Die Vorrichtung ist insbesondere für Zweikomponenten-side-by-side-Kartuschensysteme vorgesehen. Ein Verfahren zum Synchronisieren von Fluidströmen sowie die Verwendung der Vorrichtung sind ebenfalls Gegenstand der Erfindung.

Unter einem "idealen Fluid" wird insbesondere eine Substanz verstanden, die einer beliebig langsamen Scherung keinen Widerstand entgegensetzt. Nachfolgend fallen unter den Begriff "Fluid" vor allem inkompressible Fluide. Unter dem Begriff "Fluidstrom" wird ein bewegtes, fließendes Fluid verstanden. 2

Bevorzugt werden weiterhin unter dem Begriff "Fluid" auch fließfähige Pasten verstanden. Eine Paste ist ein Feststoff-Flüssigkeitsgemisch (Suspension) mit einem hohen Gehalt an Feststoffen.

Pastenförmige Zweikomponentensysteme sind in der Technik weit verbreitet und werden für Dichtungen und Klebstoffe in großem Umfang hergestellt und in Industrie, Handwerk und im Heimwerkerbereich eingesetzt. Daneben sind pastenförmige Zweikomponentensysteme im Bereich der Dentaltechnik üblich. In letzter Zeit wurden auch Versuche unternommen, pastenförmige Zweikomponenten-Polymethylmethacrylat-Knochenzement-Systeme zu entwickeln, wie beispielsweise aus der DE 10 2007 050 762 B3 bekannt. Gegenwärtig sind hierfür so genannte side-by-side-Kartuschen und Koaxial-Kartuschen technisch üblich.

Bei all diesen Zweikomponentensystemen ist das grundlegende Prinzip gleich, dass in jeweils einer Paste ein Initiator und in der anderen Paste ein Beschleuniger oder Härter enthalten ist. Die Pasten sind üblicherweise getrennt in separaten Kartuschen gelagert. Aus diesen Kartuschen werden die Pasten durch darin angebrachte Förderkolben in Richtung Kartuschenkopf durch Einwirkung von Stößeln gedrückt. Es kommt dabei darauf an, dass die Pasten in dem vorbestimmten Volumenverhältnis aus den Kartuschen in statische Mischer gepresst werden, die jeweils am Kartuschenkopf angebracht sind. In den statischen Mischern vermischen sich die Pasten und die Aushärtungsreaktion setzt ein. Für die Qualität des ausgehärteten Pastenmaterials ist es wesentlich, dass das Volumenverhältnis der Pasten zueinander eingehalten wird. Am häufigsten liegt das Volumenverhältnis der Pasten im Bereich 1:1 bis 1:10. Das Volumenverhältnis wird einerseits durch die Dimensionierung der Kartuschen gewährleist und andererseits durch den synchronen Vortrieb der Förderkolben in den Kartuschen. Technisch wird dieser synchrone Vortrieb dadurch vorgenommen, dass die Förderkolben miteinander verbunden sind. Bei den meisten Zweikomponentensystemen sind die Förderkolben mit einer Zahnstange verbunden, die entweder durch Handkraft über Bedienhebel oder durch Motoren in Richtung des Kartuschenkopfs bewegt wird.

Daneben ist es auch möglich, Kartuschen mit Druckluft auszupressen. Dabei befindet sich in dem Kartuschensystem ein Förderkolben, der an der Außenseite zwei Stößel besitzt. Der Förderkolben wird beim Auspressen mit Druckluft beaufschlagt, wodurch sich dieser in Richtung Kartuschenkopf bewegt. Die an der Außenseite angebrachten Stößel drücken gleichzeitig auf die Kolben der Kartuschen. Weil beide Stößel mit dem Förderkolben verbunden sind, können sich diese nur synchron nach vorne in Richtung Kartuschenkopf bewegen.

Die Synchronisierung des Austrags ist deshalb wichtig, weil die Pasten üblicherweise nicht die exakt gleiche Viskosität besitzen. Deshalb würde eine einfache Druckbeaufschlagung mit Druckluft auf nicht synchronisierte Kolben dazu führen, dass bei der niedrig viskoseren Paste der Förderkolben schneller in Richtung Kartuschenkopf gedrückt würde als bei der im Vergleich höher viskosen Paste. Dadurch würde das vorgegebene Volumenverhältnis verändert. Das Ergebnis wäre ein nicht optimal ausgehärtetes Pastenmaterial.

Die direkte Beaufschlagung mit komprimiertem Gas aus Druckluftleitungen oder auch aus konventionellen Druckgaspatronen, wie zum Beispiel Kohlendioxid-Patronen, hätte die Vorteile, dass sehr große Kräfte auf die Förderkolben einwirken würden, wodurch auch hoch viskose Pasten ausgepresst werden könnten, und dass der Auspressdruck und damit die Auspressgeschwindigkeit durch einfache Ventile gesteuert werden könnten, ohne das mechanische Vorrichtungen, wie Zahnstangen oder Getriebe, notwendig sind.

Besonders vorteilhaft wäre es für Anwendungen im OP-Bereich, wenn mit Druckgas ein Kartuschensystem / ein Applikator mit einem möglichst kleinen Volumen und mit einer maximalen Auspresskraft verwendet werden könnte, der eine schnelle und sichere Applikation von pastenförmigen Zweikomponenten-Polymethylmethacrylat-Knochenzementen ermöglichen würde. Dazu wäre es sinnvoll, wenn der Gasdruck direkt auf die Förderkolben in den Kartuschen einwirken könnte, ohne dass eine großvolumige Synchronisierung in Form von Förderkolben mit Stößeln notwendig wäre.

Ein gattungsgemäßes Kartuschensystem mit zwei Kartuschen und zwei miteinander verbundenen Förderkolben ist aus der US 4,260,077 A bekannt. Das Kartuschensystem ist im Prinzip wie zwei direkt nebeneinander liegende Spritzen aufgebaut. An der Spitze des Kartuschensystems ist eine gemeinsame Auslassöffnung für beide Kartuscheninhalte vorgesehen. Beim Auspressen der Inhalte werden die Förderkolben so weit in die Kartuschen gedrückt, bis die vorderen Enden der Förderkolben an die Kartuschenköpfe stoßen.

Ein weiteres Kartuschensystem ist in DE 299 23 938 U1 beschrieben, bei dem zwei parallel zueinander angeordnete Kartuschen zu mischenden Substanzen enthalten. Die Substanze liegen in Schlauchbeuteln vor, die vor der Nutzung durch zwei Anstechröhrchen aufgestochen werden. Die beiden Anstechröhrchen münden in einem Mischraum, wo die Substanzen vermischt werden.

Die GB 2 064 664 A offenbart ein Kartuschensystem zum Mischen und Applizieren eines medizinisches Zements aus zwei Fluiden, wobei bei zwei nebeneinander angeordneten Kartuschen vorne jeweils eine Kartuschenöffnung vorgesehen ist, durch die der Inhalt der Kartuschen ausgetrieben werden kann.

Zusammenfassend kann festgestellt werden, dass der grundlegende Nachteil der bisher bekannten Kartuschensysteme beziehungsweise Applikatorensysteme für pastenförmige Mehrkomponentensysteme darin besteht, dass durch die zur Synchronisierung des Pastenaustrags miteinander verbundenen Förderkolben beziehungsweise Stößel der Platzbedarf der Applikatoren sehr groß ist, weil die verbundenen Förderkolben mindestens genauso lang sein müssen, wie die Kartuschen, um einen weitgehenden Austrag der Pasten aus den Kartuschen durch Bewegung der Förderkolben in Richtung Kartuschenkopf zu ermöglichen. Die-Förderkolben müssen bis zum Kartuschenkopf bewegt werden, damit die gesamte im Kartuschenvolumen enthaltende Paste in den statischen Mischer gedrückt werden kann.

Die Aufgabe der Erfindung besteht also darin, ein Verfahren und eine Vorrichtung zum Synchronisieren und Vermischen von pastenförmigen Fluiden zu entwickeln, die es erlauben, den Austrag von Pasten zu synchronisieren, so dass durch einfaches Beaufschlagen der Förderkolben in den Kartuschen mit komprimiertem Gas Pasten mit unterschiedlicher Viskosität in einem vorbestimmten Volumenverhältnis aus den Kartuschen in statische Mischer gepresst werden können. Das Kartuschensystem sollte ein möglichst kleines Volumen besitzen. Insbesondere soll die Synchronisierungsvorrichtung eine Länge deutlich kleiner als die Kartuschenlänge haben.

Die Aufgabe der Erfindung wird dadurch gelöst, dass am Kartuschenkopf eine Vorrichtung zum Regeln der Fluidströme aus den Kartuschen angeordnet ist, umfassend zumindest zwei Flügel und zumindest zwei Öffnungen, die zumindest bereichsweise wenigstens zwei Kartuschenöffnungen überdecken, wobei die Vorrichtung drehbar oder verschiebbar auf dem Kartuschenkopf angeordnet ist, wobei an zumindest zwei Öffnungen zumindest jeweils ein Flügel angeordnet ist, der zumindest bereichsweise über die Öffnung ragt, so dass eine Strömung durch die Öffnungen über die Flügel eine Kraft parallel zur Fläche der Öffnungen auf die Vorrichtung bewirkt, die zu einer Verschiebung oder Drehung der Vorrichtung gegen den Kartuschenkopf führt, bei der sich die Fläche der durch die Vorrichtung abgedeckten Kartuschenöffnungen verändert, und dass am Kartuschenkopf ein Gehäuse umfassend die Austragsöffnung angeordnet ist, in dem die Vorrichtung drehbar oder verschiebbar angeordnet ist.

Flügel sind im Sinne der vorliegenden Erfindung alle einen Fluidstrom umleitenden Flächen, die eine Kraft an die Vorrichtung weiterleiten, mit der diese drehbar oder verschiebbar ist.

Dabei kann vorgesehen sein, dass die beiden Kartuschen zylindrische Zweikomponenten-*side-by*-*side*-Kartuschen sind.

Dabei kann wiederum vorgesehen sein, dass die Vorrichtung um eine Achse parallel zur Kartuschenachse drehbar oder in einer Richtung senkrecht zur Kartuschenachse verschiebbar angeordnet ist.

Erfindungsgemäße Kartuschensysteme können sich auch dadurch auszeichnen, dass der Kartuschenkopf als plane Fläche ausgebildet ist.

Dabei kann vorgesehen sein, dass die Vorrichtung eine plane, insbesondere kreisförmige Scheibe mit zumindest zwei Öffnungen und zumindest zwei an den Öffnungen angeordneten Flügeln ist.

Es kann auch erfindungsgemäß vorgesehen sein, dass die Kartuschenöffnungen die Innenräume der Kartuschen mit der Oberseite des Kartuschenkopfs verbinden, auf der die Vorrichtung angeordnet ist.

Bei den erfindungsgemäßen Kartuschensystemen kann vorgesehen sein, dass zwei Kartuschenöffnungen im Kartuschenkopf und zwei Öffnungen in der Vorrichtung vorgesehen sind, wobei die Öffnungen die Kartuschenöffnungen zumindest bereichsweise überdecken.

Besonders vorteilhaft ist es, wenn die Flügel einen Neigungswinkel von 1° bis 89° zu den Öffnungen aufweisen, vorzugsweise 30° bis 60° und besonders bevorzugt 40° bis 50°.

Es kann auch vorgesehen sein, dass die Flügel flache Scheiben, insbesondere Platten, sind.

Eine Ausgestaltung der Erfindung sieht vor, dass die Vorrichtung verschiebbar am Kartuschenkopf gelagert ist und die Flügel bezogen auf die Verschiebungsrichtung in entgegengesetzte Richtungen geneigt sind, wobei die aufgrund einer Fluidströmung durch die Öffnungen auf die Flügel einwirkenden Kräfte bezogen auf die verschiebbare Vorrichtung entgegengesetzt gerichtet sind.

Bei einer alternativen Ausgestaltung des erfindungsgemäßen Kartuschensystems ist vorgesehen, dass die Vorrichtung drehbar am Kartuschenkopf gelagert ist und die Flügel bezogen auf die Drehachse in entgegengesetzte Richtungen geneigt sind, wobei die aufgrund einer Fluidströmung durch die Öffnungen auf die Flügel einwirkenden Drehmomente bezogen auf die Drehachse der Vorrichtung entgegengesetzt gerichtet sind.

Es kann auch vorgesehen sein, dass das Gehäuse einen Innendurchmesser größer gleich der Vorrichtung hat und der Abstand der Vorrichtung zur oberen Innenseite des Gehäuses größer ist als die Senkrechte von der Außenseite der Flügel zur Oberfläche der Vorrichtung.

Besonders vorteilhaft sind Kartuschensysteme, bei denen vorgesehen ist, dass das Gehäuse an der Innenseite eine Führung für die Vorrichtung hat.

Es kann vorgesehen sein, dass an der Austragsöffnung ein erstes Befestigungsmittel, insbesondere ein Gewinde für ein Austragsrohr, angeordnet ist.

Vorteilhafte Kartuschensysteme können auch vorsehen, dass die Kartuschen an den dem Kartuschenkopf gegenüberliegenden Seiten durch jeweils einen Förderkolben zum Austreiben von Ausgangskomponenten des Mischguts aus den Kartuschen begrenzt sind, wobei die Förderkolben beweglich im Innenraum der Kartuschen angeordnet sind.

Es kann ferner vorgesehen sein, dass an der Austragsöffnung ein Austragsrohr umfassend einen statischen Mischer angeordnet ist.

Es ist besonders vorteilhaft, wenn an dem Gehäuse ein zweites Befestigungsmittel, insbesondere ein Innengewinde, angeordnet ist, mit dem das Gehäuse an einem an den Kartuschen angeordneten dritten Befestigungsmittel, insbesondere einem Außengewinde, vorzugsweise lösbar zu befestigen ist.

Auch kann vorgesehen sein, dass die Vorrichtung fluiddicht mit dem Kartuschenkopf abschließt, so dass zumindest zwei Kartuschenöffnungen mit zumindest zwei darüber angeordneten Öffnungen der Vorrichtung zumindest zwei durchgehende, fluiddichte Verbindungen vom Innenraum der Kartuschen in den Innenraum des Gehäuses bilden.

Gemäß einer bevorzugten Ausführungsform kann vorgesehen sein, dass die Kartuschen durch Drehen oder Verschieben der Vorrichtung verschließbar und/oder öffenbar sind.

Mit der Erfindung wird auch eine Vorrichtung für ein solches Kartuschensystem zur Verfügung gestellt, die drehbar oder verschiebbar auf einem Kartuschenkopf eines Kartuschensystems anordbar ist, mit wenigstens zwei Öffnungen (50, 150, 250), die auf dem Kartuschenkopf (20,120) derart verschiebbar sind, dass sie zumindest bereichsweise zwei Kartuschenöffnungen (30, 130, 230) des Kartuschensystems überdecken, wobei an zumindest zwei Öffnungen (50, 150, 250) zumindest jeweils ein Flügel (60, 160, 260) angeordnet ist, der zumindest bereichsweise über die Öffnung (50, 150, 250) ragt.

Die Aufgabe der Erfindung wird auch gelöst durch ein Verfahren zur Synchronisierung von Fluidströmen mit einem solchen Kartuschensystem oder mit einer solchen Vorrichtung, wobei zwei Fluidströme durch die Kartuschenöffnungen und durch die Öffnungen der Vorrichtung gedrückt werden, die Fluidströme durch die Flügel umgeleitet werden, bei ungleicher Viskosität der Fluide infolge der unterschiedlichen Strömungsgeschwindigkeit eine unterschiedliche Kraft auf die Flügel ausgeübt wird, wobei dadurch die Vorrichtung gedreht oder verschoben wird, wobei die Öffnungen gegenüber den Kartuschenöffnungen verschoben werden und dadurch die eine Durchtrittsfläche, die durch eine Kartuschenöffnung und die darüber liegende Öffnung begrenzt wird, bei dem niedrig viskoseren Fluid verkleinert wird und die andere Durchtrittsfläche bei dem höher viskosen Fluid vergrößert wird, bis die auf die Flügel einwirkenden Kräfte durch die Veränderung der Fluidströme durch die Durchtrittsflächen gleich groß werden und die Drehbewegung oder die Verschiebung der Vorrichtung gestoppt wird.

Dabei kann vorgesehen sein, dass die Fluidströme, nachdem sie durch die Öffnungen gedrückt werden, in das Gehäuse gedrückt werden und dann durch die Austragsöffnung aus dem Gehäuse heraus gedrückt werden.

Schließlich wird mit der Erfindung noch ein Verfahren zum Mischen eines Mischguts mit einem solchen Kartuschensystem vorgeschlagen, bei dem das Kartuschensystem zum Mischen von strömenden pastenförmigen Klebstoffen, pastenförmigen Dichtstoffen, pastenförmigen Lebensmitteln, pastenförmigen Dentalmaterialien, pastenförmigen anorganischen Knochenzementen und/oder pastenförmigen Polymethylmethacrylat-Knochenzementen verwendet wird, insbesondere unter Verwendung eines solchen Verfahrens.

Dabei kann vorgesehen sein, dass das gemischte Mischgut appliziert wird.

Der Erfindung liegt die überraschende Erkenntnis zugrunde, dass es durch eine einfache Vorrichtung umfassend Öffnungen und Flügel an den Öffnungen, die auf einem Kartuschenkopf zweier Kartuschen mit jeweils zumindest einer Kartuschenöffnung im Kartuschenkopf angeordnet ist, gelingt, die Fluidströme durch die übereinander angeordneten Kartuschenöffnungen und Öffnungen in der Vorrichtung durch eine Drehung oder Verschiebung der Vorrichtung auf dem Kartuschenkopf zu steuern, indem die Drehung oder Verschiebung zu einer Veränderung der für die Fluidströme effektiven durchströmten Fläche führt. Die effektive Fläche für das dünnflüssigere Medium verkleinert sich dabei relativ zur effektiven Fläche für das zähflüssigere Medium. Dabei kann sich die effektive Fläche für das dünnflüssige Medium verkleinern und/oder die effektive Fläche für das zähflüssigere Medium vergrößern. Hierdurch werden die Volumenströme durch die Veränderung der effektiven Flächen verkleinert, beziehungsweise vergrößert. Anders ausgedrückt wird der Strömungswiderstand für die beiden Fluide dadurch angepasst, dass die effektive Öffnungsfläche an deren Zähigkeit angepasst wird.

Die Vorrichtung wird dadurch angetrieben, das heißt gedreht oder verschoben, dass die Fluidströme auf die Flügel prallen und dort abgelenkt werden. Dieses Umleiten der Fluidströme bewirkt eine Kraft, die von den Flügeln auf die Vorrichtung weitergegeben wird und die proportional dem Massestrom, beziehungsweise dem Impuls der Fluide, ist. Die Vorrichtung kann dieser Kraft durch eine Bewegung (Drehung oder Verschieben) ausweichen. Bei geeigneter Anordnung der Flügel führt dies zu einer Drehung oder zu einer Verschiebung der Vorrichtung, was wiederum die effektive Öffnung verändert, wodurch sich die Volumenströme der Fluide ändern und wodurch sich die auf die Flügel wirkenden Kräfte ändern. Die Bewegung endet, sobald ein Gleichgewicht der beiden auf die Flügel wirkenden Kräfte erreicht ist. Bei geeigneter Geometrie der Kartuschenöffnungen und bei geeigneter Geometrie und Lagerreibung der Vorrichtung, ihrer Öffnungen und Flügel, kann ein gewünschtes Mischungsverhältnis unabhängig von der Viskosität der zu mischenden Fluide erzielt werden.

Damit wird erreicht, dass sich schnell das gewünschte Mischungsverhältnis einstellt. Bei geeigneter Anpassung der Geometrie und der Reibungsverluste der Vorrichtung ist es also mit einem erfindungsgemäßen Kartuschensystem und mit den erfindungsgemäßen Verfahren leicht möglich, dass stets das gleiche Mischungsverhältnis unabhängig von der Viskosität der beiden Fluide erzielt wird.

Erfindungsgemäße Kartuschensysteme können zum Synchronisieren und Vermischen von strömenden pastenförmigen Klebstoffen, pastenförmigen Dichtstoffen, pastenförmigen Lebensmitteln, pastenförmigen Dentalmaterialien, pastenförmigen anorganischen Knochenzementen und pastenförmigen Polymethylmethacrylat-Knochenzementen verwendet werden.

Es ist auch vorteilhaft, einen Satz von Vorrichtungen mit unterschiedlichen Flügelgeometrien, Öffnungsgrößen und/oder Öffnungsformen für ein erfindungsgemäßes Kartuschensystem bereitzustellen, mit denen unterschiedliche vorgegebene Mischungsverhältnisse von zwei Fluiden, insbesondere medizinischen Zementen, in zwei Kartuschen einstellbar sind.

Im Folgenden werden Ausführungsbeispiele der Erfindung anhand von acht schematisch dargestellten Figuren erläutert, ohne jedoch dabei die Erfindung zu beschränken. Dabei zeigt:
- Figur 1:: eine schematische Querschnittansicht eines erfindungsgemäßen Kartuschensystems;
- Figur 2:: eine schematische Aufsicht auf einen Kartuschenkopf eines zweiten erfindungsgemäßen Kartuschensystems;
- Figur 3:: eine schematische Aufsicht auf einen Kartuschenkopf des erfindungsgemäßen Kartuschensystems nach Figur 1 mit drehbarer Scheibe;
- Figur 4:: eine schematische Aufsicht auf die Oberseite einer drehbaren Scheibe für ein erfindungsgemäßes Kartuschensystem nach Figur 3;
- Figur 5:: eine schematische Seitenansicht auf die drehbare Scheibe nach Figur 3;
- Figur 6:: eine schematische Aufsicht auf die Unterseite einer drehbaren Scheibe nach Figur 3;
- Figur 7:: eine schematische Aufsicht auf die Oberseite einer verschiebbaren Vorrichtung für ein drittes erfindungsgemäßes Kartuschensystem; und
- Figur 8:: eine schematische Seitenansicht auf die drehbare Scheibe nach Figur 7.

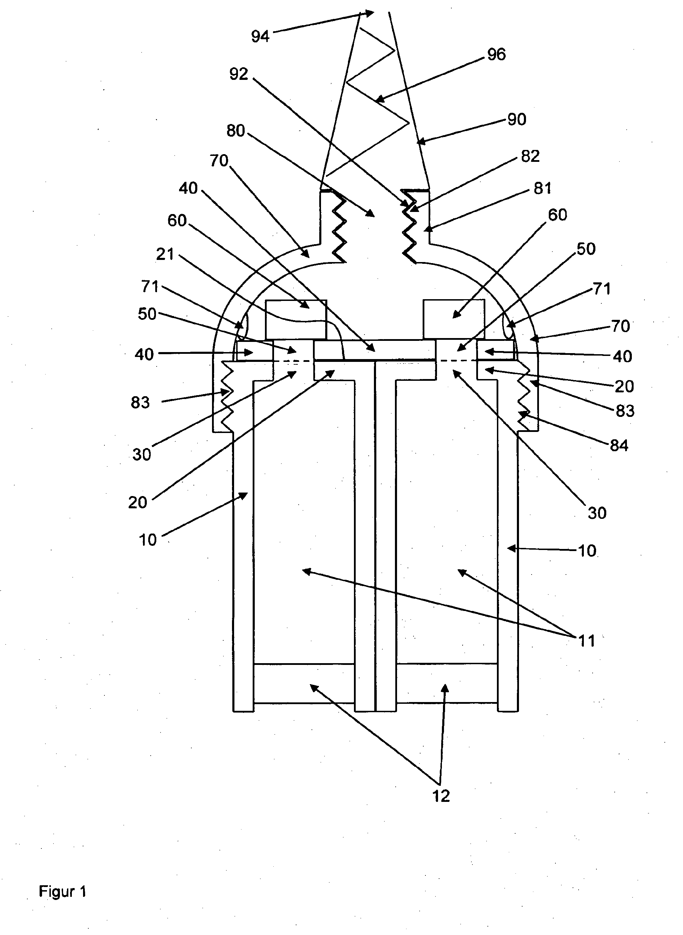

Figur 1 zeigt eine schematische Querschnittansicht eines erfindungsgemäßen Kartuschensystems. Das Kartuschensystem umfasst zwei zylindrische Kartuschen (10), die jeweils einen Innenraum (11) umfassen, in denen die zu mischenden Mischgüter (hier Pasten, insbesondere medizinische Zemente) enthalten sind. Bodenseitig sind in den Kartuschen (10) zwei zylindrische Förderkolben (12) angeordnet, die mit den Innenwänden der Kartuschen (10) die Innenräume (11) dicht abschließen. Die Förderkolben (12) sind in Richtung der Zylinderachsen der Kartuschen (10) verschiebbar angeordnet.

An der den Förderkolben (12) gegenüberliegenden Seite der Kartuschen (10) sind beide Kartuschen durch einen Kartuschenkopf (20) abgeschlossen. Der Kartuschenkopf (20) ist auf der Oberseite durch eine Kartuschenkopfaußenfläche (21) begrenzt. Diese Kartuschenkopfaußenfläche (21) ist vorzugsweise eine plane Fläche, kann aber auch gewölbt oder anders geformt sein. Im Kartuschenkopf (20) befinden sich zwei Kartuschenöffnungen (30), die jeweils einen der Innenräume (11) der Kartuschen (10) kopfseitig öffnen. Wenn ein Gasdruck (zum Beispiel Druckluft oder das Gas aus einer CO₂-Patrone) bodenseitig an den Förderkolben (12) angelegt wird, werden die Förderkolben (12) in Richtung des Kartuschenkopfs (20) getrieben. Dadurch wird der Inhalt der Kartuschen (10) durch die Kartuschenöffnungen (30) gepresst.

Oberhalb des Kartuschenkopfs (20) ist eine Vorrichtung (40) in Form einer Scheibe angeordnet, die ebenfalls zwei Öffnungen (50) umfasst. Die Vorrichtung (40) ist drehbar oder verschiebbar auf dem Kartuschenkopf (20) gelagert und liegt auf der Kartuschenkopfaußenfläche (21) flächig auf. Die Kartuschenkopfaußenfläche (21) ist also derart auszuformen, dass sie die gewünschte Bewegung der Vorrichtung (40) nicht verhindert oder behindert. Die beiden Öffnungen (50) überdecken die beiden Kartuschenöffnungen (30) bereichsweise, so dass diese zwei durchgehende effektive Öffnungen (30, 50) bilden. Durch eine Drehung oder ein Verschieben der Vorrichtung (40) auf dem Kartuschenkopf (20) verändern sich die Flächen, mit der die Öffnungen (50) die Kartuschenöffnungen (30) überdecken, und zwar derart, dass die eine effektive Öffnung (30, 50) größer wird, wenn die andere effektive Öffnung (30, 50) kleiner wird. An beiden Öffnungen (50) sind Flügel (60) angeordnet, die sich schräg zumindest bereichsweise über die Öffnungen erstrecken. Ein durch die Öffnungen (50) strömendes Fluid trifft auf die Flügel (60) und wird dort abgelenkt. Daraus resultiert eine Kraft auf die Vorrichtung (40), die von der Neigung und Fläche der Flügel (60) und von den Masseströmen der Fluide aus den Kartuschen (10) abhängt.

Wenn die beiden Flügel (60) entgegengesetzt geneigt sind, ist die Vorrichtung (40) in zwei Richtungen verschiebbar gelagert. Wenn die beiden Flügel (60) in die gleiche Richtung geneigt sind, ist die Vorrichtung (40) drehbar gelagert. Durch die Drehung oder die Verschiebung verschieben sich die Öffnungen (50) gegen die Kartuschenöffnungen (30) derart, dass die effektive Öffnung (30, 50), durch die ein größerer Massestrom strömt, kleiner wird, während die andere effektive Öffnung (30, 50), durch die der kleinere Massestrom fließt, größer wird. Ein Gleichgewicht wird sich dann einstellen, wenn bei gleicher Gestaltung der Flügel (60) beide Masseströme gleich groß sind. Bei unterschiedlicher Gestaltung, insbesondere der Fläche der Flügel (60) können andere Mischungsverhältnisse der Fluide im Gleichgewichtszustand erreicht werden.

In jedem Fall strömen die Fluide schließlich in einen durch eine Abdeckung (70) abgeschlossenen Raum, in dem sich die Fluide mischen. An den Innenwänden der Abdeckung (70) ist eine Führung (71) angeordnet, die die Vorrichtung (40) in Position hält und auch die Beweglichkeit der Vorrichtung (40) gezielt einschränken kann. Eine drehbare Lagerung der Vorrichtung (40) kann durch einen zentrischen Zapfen (nicht gezeigt) am Kartuschenkopf (20) und/oder an der Vorrichtung (40) selbst unterstützt werden. Für eine Verschiebbarkeit der Vorrichtung (40) können Schienen (nicht gezeigt) oder Vorsprünge (nicht gezeigt) am Kartuschenkopf (20), der Abdeckung (70) und/oder an der Vorrichtung (40) vorgesehen sein.
An der Oberseite der Abdeckung (70) ist eine Austragsöffnung (80) für das gemischte Mischgut, beziehungsweise die gemischten Fluide, vorgesehen. Die Austragsöffnung ist in einem zylindrischen Stutzen (81) mit kreisförmiger Grundfläche angeordnet. Auf der Innenseite des Stutzens (81) ist ein Innengewinde (82) vorgesehen. An der Unterseite der Abdeckung (70) ist ein Befestigungsmittel (83), das beispielsweise eine Rastung oder ein Innengewinde sein kann, angeordnet. Mit dem Befestigungsmittel (83) kann die Abdeckung (70) an den beiden Kartuschen (10) befestigt werden. Zu diesem Zweck umfassen die Kartuschen (10) ein Befestigungsmittel (84), beispielsweise eine Gegenrastung oder ein Außengewinde, das in das Befestigungsmittel (83) der Abdeckung (70) greift.

Das Innengewinde (82) des Stutzens (81) dient dazu, daran ein Austragsrohr (90) zu befestigen, dass ein Außengewinde (92) umfasst. Anstatt eines Innengewindes (82) und eines Außengewindes (92) kann das Austragsrohr (90) auch mit anderen Befestigungsmitteln (82, 90) an der Austragsöffnung (80) befestigt werden. Hierzu kommen insbesondere Rastungen, Schnappungen, Bajonettverschlüsse, Klebeverschlüsse und einfache Steckverbindungen in Frage, die einfach aufgebaut werden können, aber auch komplexere Befestigungsmittel wie Flansche sind möglich. Ebenso gut kann das Austragsrohr (90) auch fest mit der Abdeckung (70), beispielsweise einteilig, verbunden sein. An der oberen Spitze des Austragsrohrs (90) ist eine Austragsrohrspitze (94) angeordnet, mit der das Mischgut applizierbar ist. Zur besseren Durchmischung der Fluide ist in dem Austragsrohr (90) ein statischer Mischer (96) angeordnet.

Die im Austragsrohr (90) und der Austragsöffnung (80) gebildeten Öffnungen für das Mischgut sind bevorzugt größer als die beiden Öffnungen (50) zusammen und die beiden Kartuschenöffnungen (30) zusammen. Dies dient dazu, den größten Widerstand für die Strömung der Fluide an den Flügeln (60) der Vorrichtung (40) zu generieren und deren Wirkung nicht durch einen Staudruck zu mindern.

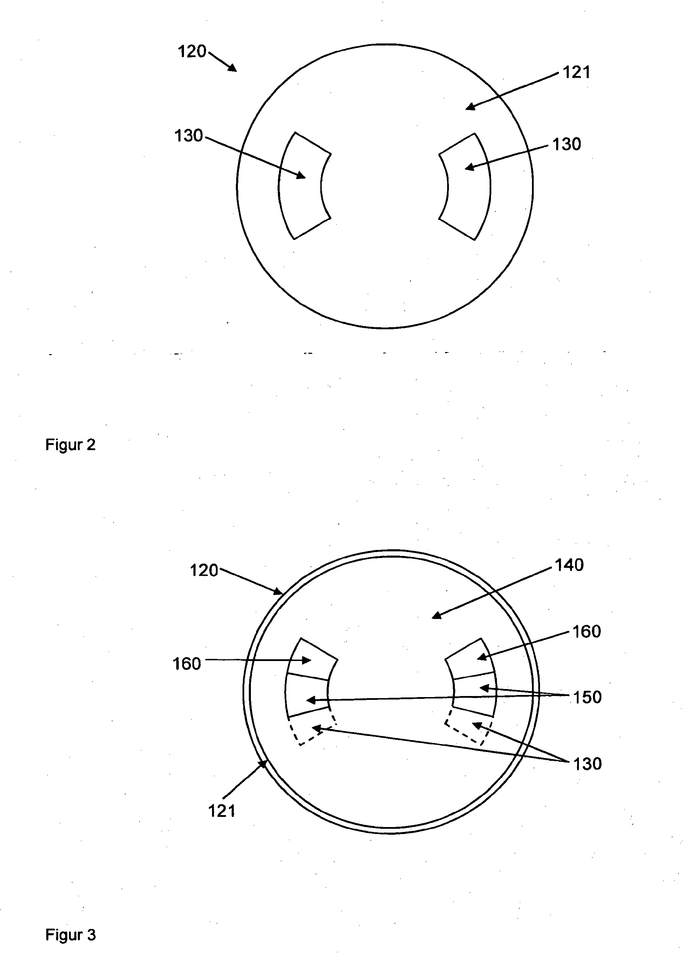

Figur 2 zeigt eine schematische Aufsicht auf ein erfindungsgemäßes, zylindrisches Kartuschensystem. Ein Kartuschenkopf (120) des Kartuschensystems ist durch eine Kartuschenkopfaußenfläche (121) in Richtung des Betrachters begrenzt. Im Kartuschenkopf (120) sind zwei Kartuschenöffnungen (130) angeordnet. Jede der Kartuschenöffnungen (130) ist mit dem Inneren einer darunter liegenden Kartusche (nicht gezeigt) verbunden. Die beiden Kartuschenöffnungen (130) sind mit gleichem Radius auf einer Kreisbahn angeordnet und stellen Ringsegmente dar.

Figur 3 zeigt eine schematische Aufsicht auf das erfindungsgemäße, zylindrische Kartuschensystem nach Figur 2, wobei auf dem Kartuschenkopf (120) eine drehbar gelagerte Vorrichtung (140) zum Steuern des Volumenstroms durch die beiden Kartuschenöffnungen (130) angeordnet ist. Zu diesem Zweck umfasst die Vorrichtung (140), die als Kreisscheibe ausgebildet ist, zwei Öffnungen (150), die bereichsweise über den Kartuschenöffnungen (130) von zwei Kartuschen (nicht zu erkennen, da durch den Kartuschenkopf (120) verdeckt) angeordnet sind. Bezogen auf die Drehachse der Vorrichtung (140), die im Zentrum der Kreisscheibe angeordnet ist, sind zwei Flügel (160) entgegengesetzt orientiert an den Öffnungen (150) angeordnet. Unter den Flügeln (160) setzen sich die Öffnungen (150) und die Kartuschenöffnungen (130) fort.

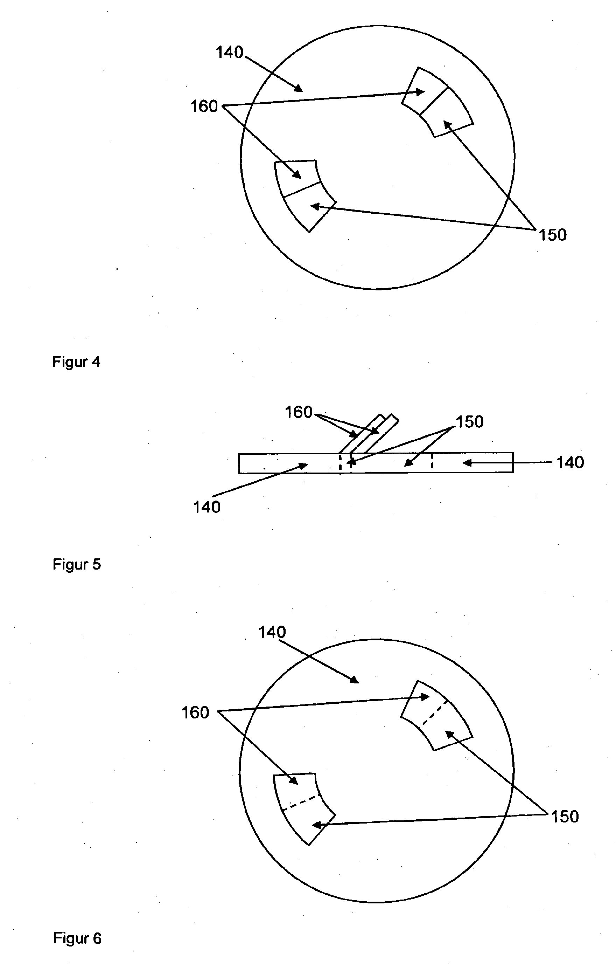

Figur 4 zeigt die Vorrichtung (140) nach Figur 3 in schematischer Aufsicht. Wie zu Figur 3 ausgeführt, sind, bezogen auf die Drehachse der Vorrichtung (140), die zwei Flügel (160) entgegengesetzt orientiert an den Öffnungen (150) angeordnet. Unter den Flügeln (160) setzen sich die Öffnungen (150) fort. Die geraden, linken, oberen Kanten der beiden dargestellten Ringsegmente definieren sowohl den Rand der Öffnungen (150), als auch den Beginn der Flügel (160). Die Flügel (160) sind an diesen geraden Kanten an der Vorrichtung (140) befestigt und ragen mit den gegenüberliegenden geraden Kanten der Flügel (160) in Richtung des Betrachters aus dem Bild heraus.

Die Neigung der Flügel (160) ist besser in Figur 5 zu erkennen, in der eine schematische Seitenansicht der Vorrichtung (140), die in den Figuren 3 und 4 gezeigt ist, dargestellt ist. Die Flügel (160) sind bezogen auf die Öffnungen (150) und in diesem Fall auch bezogen auf die kreisscheibenförmige, ebene Vorrichtung (140) um einen Winkel von 45° geneigt. Eine Strömung eines Fluids durch die Öffnungen (150) von unten führt dazu, dass der Fluidstrom bereichsweise durch die Flügel (160) nach rechts umgeleitet wird.

Figur 6 zeigt die in den Figuren 3, 4 und 5 dargestellte Vorrichtung (140) in einer Ansicht von unten. Die Flügel (160) sind durch die Öffnungen (150) hindurch zu erkennen. Die gestrichelte Kante der Flügel (160) liegt vom Betrachter aus gesehen am weitesten unterhalb der Vorrichtung (140) und ist nur aus diesem Grund gestrichelt dargestellt, auch wenn sie Ansonsten klar durch die Öffnungen (150) hindurch sichtbar ist.

Bezogen auf die Drehachse der Vorrichtung (140) sind die beiden Flügel (160) in entgegengesetzte Richtungen geneigt. Das bedeutet, dass bei einer Drehung der Vorrichtung (140) im Uhrzeigersinn bei der in Figur 3 gezeigten Aufsicht der rechte obere Flügel (160) in Richtung der Drehung geneigt ist, während bei einer Drehung entgegen des Uhrzeigersinns der linke untere Flügel (160) in Richtung der Drehung geneigt ist. Ein unterschiedlicher Massestrom durch die Öffnungen (150) führt durch die Umleitung der Ströme an den Flügeln (160) dazu, dass die Vorrichtung (140) gedreht wird, da die über die Flügel (160) angreifenden Drehmomente entgegengesetzt gerichtet sind.

Es ist somit beispielsweise ein Kartuschensystem für Zweikompenenten-*side*-*by*-*side-*Kartuschen (10) erfindungsgemäß, das dadurch charakterisiert ist, dass
a) der Kartuschenkopf (20, 120) als plane Fläche (21, 121) mit zwei Kartuschenöffnungen (30, 130) ausgebildet ist, wobei die Kartuschenöffnungen (30, 130) den Innenraum (11) der Kartuschen (10) mit der Oberseite der planen Fläche (21, 121) verbinden,
b) eine plane Scheibe (40, 140) auf der planen Fläche (21, 121) des Kartuschenkopfs (20, 120) angeordnet ist,
c) die plane Scheibe (40, 140) mindestens zwei Öffnungen (50, 150) enthält, die eine kleinere Fläche haben als die Kartuschenöffnungen (30, 130);
d) die Scheibe (40, 140) drehbar zur Kartuschensystemachse angeordnet ist,
e) die Scheibe (40, 140) so drehbar angeordnet ist, dass die Öffnungen (50, 150) über den Kartuschenöffnungen (30, 130) liegen,
f) auf der Scheibe (40, 140) bei jeder Öffnung (50, 150) mindestens zwei zur Scheibe (40, 140) hin geneigte Flügel (60, 160) angeordnet sind, wobei der Neigungswinkel der Flügel (60, 160) im Bereich von 1° bis 90° zur Scheibe (40, 140) ist
g) die Flügel (60, 160) benachbart zu den Öffnungen (50, 150) angeordnet sind,
h) die Flügel (60, 160) mindestens einen Teil der Öffnungen (50, 150) abdecken,
i) der Öffnungswinkel der Flügel (60, 160) in die gleiche Richtung zeigt,
j) die Scheibe (40, 140) mit den Öffnungen (50, 150) und den Flügeln (60, 160) durch ein Gehäuse (70) abgedeckt ist, wobei das Gehäuse (70) einen Innendurchmesser größer gleich der Scheibe (40, 140) hat und wobei der Abstand der Scheibe (40, 140) zur oberen Innenseite des Gehäuses (70) größer ist als die Senkrechte von der Außenseite der Flügel (60, 160) zur Oberfläche der Scheibe (40, 140),
k) das Gehäuse (70) an der Innenseite eine Führung für die Scheibe (40, 140) hat,
l) an der Oberseite des Gehäuses (70) eine Austragsöffnung (80) angeordnet ist, an der sich ein Befestigungsmittel (82) für ein Austragsrohr (90) mit einem darin angeordneten statischen Mischer (96) befindet und
m) an der Unterseite des Gehäuses (70) ein Befestigungsmittel (83) vorhanden ist, mit dem das Gehäuse (70) mit den Kartuschen (10) verbindbar ist.

Die Flügel (60, 160) können einfach durch schräg stehende Platten (60, 160) realisiert werden.

Es ist besonders vorteilhaft, wenn die Kartuschenöffnungen (30, 130) im Kartuschenkopf (20, 120) so angeordnet sind, dass die Öffnungen (50, 150) der Scheibe (40, 140) mit der Kante, an der sich die Flügel (60, 160) befinden, deckungsgleich mit jeweils einer Kante der Kartuschenöffnungen (30, 130) sind, wenn bei beiden Öffnungen (50, 150) die durch Überlappung der Querschnitte der Kartuschenöffnungen (30, 130) und Öffnungen (50, 150) gebildete Durchtrittsfläche für beide Öffnungen (30, 50, 130, 150) gleich ist. Die Kartuschenöffnungen (30, 130) haben eine größere Querschnittfläche als die Öffnungen (50, 150). Die Kartuschenöffnungen (30, 130) sind entgegengesetzt zu den Kanten der Öffnungen (50, 150), an denen die Flügel (60, 160) angeordnet sind, größer ausgebildet als die Kartuschenöffnungen (30, 130). Das bedeutet, dass bei gleicher Durchtrittsfläche zwischen den Kartuschenöffnungen (30, 130) und den Öffnungen (50, 150) immer ein Teil der Kartuschenöffnungen (30, 130) abgedeckt ist.

Die Idee der Erfindung beruht auch darauf, dass eine drehbare Scheibe (40, 140) angeordnet ist, die Öffnungen (50, 150) besitzt, die mindestens teilweise von schrägen Flügeln (60, 160) abgedeckt sind, wobei die Scheibe (40, 140) über einer planen Fläche (21, 121) angeordnet ist, die zwei Kartuschenöffnungen (30, 130) besitzt. Wenn unterschiedlich viskose Fluide, zum Beispiel durch Einwirkung von komprimiertem Gas auf die Förderkolben (12), in Richtung Kartuschenkopf (20, 120) gepresst werden, dann fließt das niedrig viskose Fluid schneller als das höher viskose Fluid. Bei dem Auftreffen und Umströmen der Fluide auf die schrägen Flügel (60, 160), wird senkrecht zur Strömungsrichtung eine zur Scheibe (40, 140) parallele Kraft auf die Flügel (60, 160) ausgeübt. Dadurch, dass die Flügel (60, 160) in die gleiche Richtung geschrägt sind, wirken die auf die Flügel (60, 160) gerichteten Kräfte gegeneinander. Bei niedrigerer Strömungsgeschwindigkeit eines Fluids gegenüber einem zweiten Fluid ist die auf den ersten Flügel (60, 160) wirkende Kraft kleiner als die Kraft, die durch das Fluid mit höherer Strömungsgeschwindigkeit auf den zweiten Flügel (60, 160) einwirkt. Dadurch wirkt auf die Scheibe (40, 140) eine resultierende Kraft, welche eine Drehbewegung der Scheibe (40, 140) verursacht. Die Kartuschenöffnungen (30, 130) des Kartuschenkopfs (20, 120) sind größer als die Öffnungen (50, 150) der Scheibe (40, 140). Dadurch wird durch das schneller fließende Fluid die Scheibe (40, 140) so gedreht, dass die Öffnung (50, 150) bei dem schnelleren Fluid sich von der Kartuschenöffnung (30, 130) der Kartuschen (10) für das schnellere Fluid wegbewegt. Dadurch wird die Durchtrittsfläche für das Fluid kleiner und es strömt weniger Fluid hindurch. Gleichzeitig wird die Öffnung (50, 150) des höher viskosen Fluids ebenfalls mit gedreht, aber in Richtung der Kartuschenöffnung (30, 130). Dadurch, dass die Kartuschenöffnungen (30, 130) größer sind als die Öffnungen (50, 150), vergrößert sich somit die Durchtrittsfläche. Es kann mehr Fluid durchfließen. Die Drehbewegung erfolgt so lange bis die Strömungsgeschwindigkeiten der beiden Fluide gleich sind beziehungsweise die damit resultierenden Kräfte gleich sind und sich dadurch aufheben. Die Strömungsgeschwindigkeit der beiden Fluide ist dann synchron.

Vorteilhaft ist auch ein Verfahren zur Synchronisierung von Fluiden, das dadurch charakterisiert ist, dass zwei Fluidströme durch die Kartuschenöffnungen (30, 130) durch die plane Fläche (21, 121) gedrückt werden. Danach fließen die Ströme durch die Öffnungen (50, 150) der Scheibe (40, 140) und drücken auf die Flügel (60, 160). Anschließend treten sie in das Gehäuse (70) ein und verlassen dann durch die Austragsöffnung (80) das Gehäuse (70), wobei bei ungleicher Viskosität der Fluide infolge der unterschiedlichen Strömungsgeschwindigkeit eine unterschiedliche Kraft auf die Flügel (60, 160) ausgeübt wird, wobei die auf die Flügel (60, 160) einwirkenden Kräfte beziehungsweise Drehmomente entgegengesetzt gerichtet sind und sich dadurch die Scheibe (40, 140) dreht. Die Öffnungen (50, 150) werden dabei gegenüber den Kartuschenöffnungen (30, 130) verschoben und dadurch wird die Durchtrittsfläche der Öffnung (50, 150) bei dem niedrig viskoseren Fluid verkleinert und die Durchtrittsfläche bei dem höher viskosen Fluid vergrößert, bis die auf die Flügel (60, 160) einwirkenden Kräfte gleich sind und sich dadurch kompensieren und dadurch die Drehbewegung der Scheibe (40, 140) gestoppt wird.

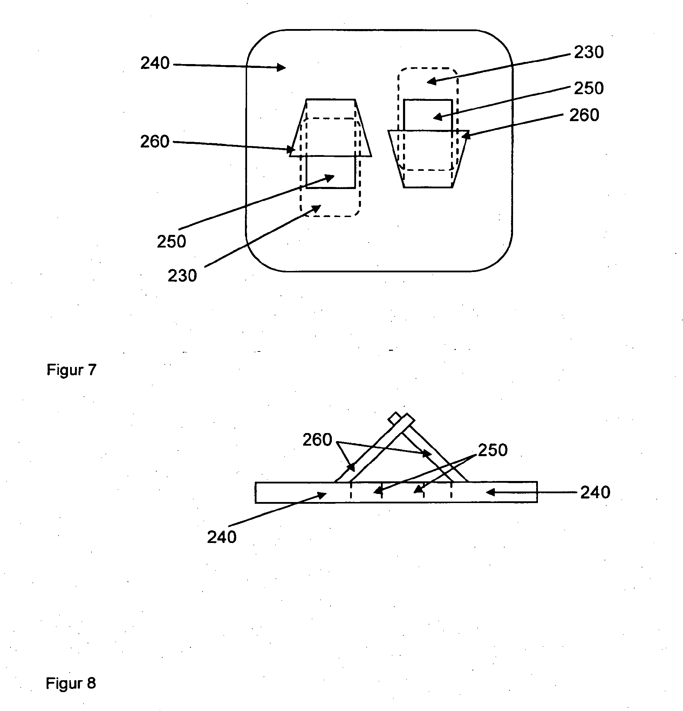

Figur 7 zeigt eine schematische Aufsicht auf zwei Kartuschenöffnungen (230) und eine darüber angeordnete, verschiebbare Vorrichtung (240) für ein erfindungsgemäßes Kartuschensystem, wie es beispielsweise in Figur 1 gezeigt ist. Figur 8 ist eine schematische Seitenansicht der Vorrichtung (240) nach Figur 7. Die Kartuschenöffnungen (230) sind in Figur 7 als gestrichelte Rechtecke mit abgerundeten Ecken dargestellt. Über den Kartuschenöffnungen (230) sind zwei Öffnungen (250) in der verschiebbaren Vorrichtung (240) vorgesehen, die die Kartuschenöffnungen (230) bereichsweise überdecken. Die Öffnungen (250) sind wiederum teilweise von Flügeln (260) überdeckt, die sich schräg über die Öffnungen (250) erstrecken. Der überdeckte Bereich der Öffnungen (250) ist in Figur 7 durch gestrichelte Linien dargestellt. Die Flügel (260) bilden eine gemeinsame Kante mit den Öffnungen (250). Die Vorrichtung (240) wird auf einem Kartuschenkopf (nicht gezeigt) verschiebbar angeordnet, so dass sie in Figur 7 von oben nach unten und umgekehrt verschiebbar ist, beziehungsweise in Figur 8 von links nach rechts und umgekehrt verschiebbar ist.

Bezogen auf diese Verschiebungsrichtung sind die Flügel (260) in entgegengesetzte Richtungen geneigt. Bezogen auf Figur 7 führt ein stärkerer Massestrom durch die linke Kartuschenöffnung (230) und damit durch die Öffnung (250) dazu, dass der durch den linken Flügel (260) nach unten abgelenkte Massestrom stärker ist als der durch den rechten Flügel (260) nach oben abgelenkte Massestrom. Die Vorrichtung (240) erfährt durch den linken Massestrom eine stärkere Kraft über den linken Flügel (260) als der rechte Flügel (260) durch den rechten Massestrom. Auf die Vorrichtung (240) wirkt somit eine resultierende Kraft, die die Vorrichtung (240) nach oben verschiebt. Durch die Verschiebung verkleinert sich der Überlapp der linken Öffnung (250) mit der linken Kartuschenöffnung (230). Dadurch wird der Volumenstrom und damit der Massestrom aus der linken Kartusche (nicht gezeigt) durch die linke Kartuschenöffnung (230) und die linke Öffnung (250) reduziert. Gleichzeitig vergrößert sich der Bereich, bei dem die rechte Öffnung (250) die rechte Kartuschenöffnung (230) überdeckt. Hierdurch werden der Volumenstrom und damit der Massestrom aus der unter der rechten Kartuschenöffnung (230) angeordneten rechten Kartusche (nicht gezeigt) vergrößert.

Sobald die Masseströme gleich sind (bei gleicher Masse der Fluide die Volumenströme gleich sind), sind die beiden auf die Flügel (260) wirkenden Kräfte gleich groß und die Vorrichtung (240) wird ihre Position nicht mehr verändern. Dadurch stellt sich auch bei einer verschiebbaren Vorrichtung (240) ein gewünschtes Mischungsverhältnis ein. Die in den Figuren 7 und 8 gezeigte Vorrichtung (240) wird ein Mischungsverhältnis von etwa 1:1 erzeugen, wenn die Dichten der Flüssigkeiten gleich groß sind. Durch eine Veränderung der Geometrie der Flügel (260) und der Größen der Öffnungen (250) und der Kartuschenöffnungen (230) können praktisch beliebige Mischungsverhältnisse erzielbar sein.

Der Unterschied zwischen den drehbaren Vorrichtungen (beispielsweise (140)) und den verschiebbaren Vorrichtungen (beispielsweise (240)) im Sinne der Erfindung ist darin zu sehen, dass bei drehbaren Vorrichtungen entgegengesetzte Drehmomente durch die Fluidströmungen erzeugt werden, während bei verschiebbaren Vorrichtungen entgegengesetzte Kräfte durch die Fluidströmungen aus den beiden Kartuschen verursacht werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 10: Kartusche
- 11: Innenraum
- 12: Förderkolben
- 20, 120: Kartuschenkopf
- 21, 121: Kartuschenkopfaußenfläche
- 30, 130, 230: Kartuschenöffnung
- 40, 140, 240: Vorrichtung / Scheibe
- 50, 150, 250: Öffnung
- 60, 160, 260: Flügel / Platte
- 70: Gehäuse
- 71: Führung
- 80: Austragsöffnung
- 81: Stutzen
- 82, 83: Befestigungsmittel / Innengewinde
- 84: Befestigungsmittel / Außengewinde
- 90: Austragsrohr
- 92: Befestigungsmittel / Außengewinde
- 94: Austragsrohrspitze
- 96: statischer Mischer

## Patentansprüche

1. Kartuschensystem zum Mischen und Applizieren zweier Fluide, insbesondere eines medizinischen Zements, umfassend zwei Kartuschen (10) und eine Austragsöffnung (80), wobei die Kartuschen (10) durch Kartuschenwände und durch einen Kartuschenkopf (20, 120) begrenzt sind, wobei im Kartuschenkopf (20, 120) wenigstens jeweils eine Kartuschenöffnung (30, 130, 230) zum Austreiben der Kartuscheninhalte aus beiden Kartuschen (10) vorgesehen ist, **dadurch gekennzeichnet, dass**
am Kartuschenkopf (20, 120) eine Vorrichtung (40, 140, 240) zum Regeln der Fluidströme aus den Kartuschen (10) angeordnet ist, umfassend zumindest zwei Flügel (60, 160, 260) und zumindest zwei Öffnungen (50, 150, 250), die zumindest bereichsweise wenigstens zwei Kartuschenöffnungen (30, 130, 230) überdecken, wobei die Vorrichtung (40, 140, 240) drehbar oder verschiebbar auf dem Kartuschenkopf (20, 120) angeordnet ist, wobei an zumindest zwei Öffnungen (50, 150, 250) zumindest jeweils ein Flügel (60, 160, 260) angeordnet ist, der zumindest bereichsweise über die Öffnung (50, 150, 250) ragt, so dass eine Strömung durch die Öffnungen (50, 150, 250) über die Flügel (60, 160, 260) eine Kraft parallel zur Fläche der Öffnungen (50, 150, 250) auf die Vorrichtung (40, 140, 240) bewirkt, die zu einer Verschiebung oder Drehung der Vorrichtung (40, 140, 240) gegen den Kartuschenkopf (20, 120) führt, bei der sich die Fläche der durch die Vorrichtung (40, 140, 240) abgedeckten Kartuschenöffnungen (30, 130, 230) verändert, und dass am Kartuschenkopf (20, 120) ein Gehäuse (70) umfassend die Austragsöffnung (80) angeordnet ist, in dem die Vorrichtung (40, 140, 240) drehbar oder verschiebbar angeordnet ist.

2. Kartuschensystem nach Anspruch 1, **dadurch gekennzeichnet, dass**
die beiden Kartuschen (10) zylindrische Zweikomponenten-side-by-side-Kartuschen (10) sind.

3. Kartuschensystem nach Anspruch 2, **dadurch gekennzeichnet, dass**
die Vorrichtung (40, 140, 240) um eine Achse parallel zur Kartuschenachse drehbar oder in einer Richtung senkrecht zur Kartuschenachse verschiebbar angeordnet ist.

4. Kartuschensystem nach einem der vorangehenden Ansprüche, **dadurch gekennzeichnet, dass**
der Kartuschenkopf (20, 120) als plane Fläche (121) ausgebildet ist.

5. Kartuschensystem nach Anspruch 4, **dadurch gekennzeichnet, dass**
die Vorrichtung (40, 140, 240) eine plane, insbesondere kreisförmige Scheibe (140, 240) mit zumindest zwei Öffnungen (50, 150, 250) und zumindest zwei an den Öffnungen angeordneten Flügeln (60, 160, 260) ist.

6. Kartuschensystem nach einem der vorangehenden Ansprüche, **dadurch gekennzeichnet, dass**
die Kartuschenöffnungen (30, 130, 230) die Innenräume (11) der Kartuschen (10) mit der Oberseite des Kartuschenkopfs (20, 120) verbinden, auf der die Vorrichtung (40, 140, 240) angeordnet ist.

7. Kartuschensystem nach einem der vorangehenden Ansprüche, **dadurch gekennzeichnet, dass**
zwei Kartuschenöffnungen (30, 130, 230) im Kartuschenkopf (20, 120) und zwei Öffnungen (50, 150, 250) in der Vorrichtung (40, 140, 240) vorgesehen sind, wobei die Öffnungen (50, 150, 250) die Kartuschenöffnungen (30, 130, 230) zumindest bereichsweise überdecken.

8. Kartuschensystem nach einem der vorangehenden Ansprüche, **dadurch gekennzeichnet, dass**
die Flügel (60, 160, 260) einen Neigungswinkel von 1° bis 89° zu den Öffnungen (50, 150, 250) aufweisen, vorzugsweise 30° bis 60° und besonders bevorzugt 40° bis 50°.

9. Kartuschensystem nach einem der vorangehenden Ansprüche, **dadurch gekennzeichnet, dass**
die Flügel (60, 160, 260) flache Scheiben, insbesondere Platten, sind.

10. Kartuschensystem nach einem der vorangehenden Ansprüche, **dadurch gekennzeichnet, dass**
die Vorrichtung (40, 240) verschiebbar am Kartuschenkopf (20) gelagert ist und die Flügel (60, 260) bezogen auf die Verschiebungsrichtung in entgegengesetzte Richtungen geneigt sind, wobei die aufgrund einer Fluidströmung durch die Öffnungen (50, 250) auf die Flügel (60, 260) einwirkenden Kräfte bezogen auf die verschiebbare Vorrichtung (40, 240) entgegengesetzt gerichtet sind.

11. Kartuschensystem nach einem der Ansprüche 1 bis 9, **dadurch gekennzeichnet, dass** die Vorrichtung (40, 140) drehbar am Kartuschenkopf (20, 120) gelagert ist und die Flügel (60, 160) bezogen auf die Drehachse in entgegengesetzte Richtungen geneigt sind, - wobei die aufgrund einer Fluidströmung durch die Öffnungen (50, 150) auf die Flügel (60, 160) einwirkenden Drehmomente bezogen auf die Drehachse der Vorrichtung (40, 140) entgegengesetzt gerichtet sind.

12. Kartuschensystem nach einem der vorangehenden Ansprüche, **dadurch gekennzeichnet, dass**
das Gehäuse (70) einen Innendurchmesser größer gleich der Vorrichtung (40, 140, 240) hat und der Abstand der Vorrichtung (40, 140, 240) zur oberen Innenseite des Gehäuses (70) größer ist als die Senkrechte von der Außenseite der Flügel (60, 160, 260) zur Oberfläche der Vorrichtung (40, 140, 240).

13. Kartuschensystem nach einem der vorangehenden Ansprüche, **dadurch gekennzeichnet, dass**
das Gehäuse (70) an der Innenseite eine Führung (71) für die Vorrichtung (40, 140, 240) hat.

14. Kartuschensystem nach einem der vorangehenden Ansprüche, **dadurch gekennzeichnet, dass**
an der Austragsöffnung (80) ein erstes Befestigungsmittel (82), insbesondere ein Gewinde für ein Austragsrohr (90), angeordnet ist.

15. Kartuschensystem nach einem der vorangehenden Ansprüche, **dadurch gekennzeichnet, dass**
die Kartuschen (10) an den dem Kartuschenkopf (20, 120) gegenüberliegenden Seiten durch jeweils einen Förderkolben (12) zum Austreiben von Ausgangskomponenten des Mischguts aus den Kartuschen (10) begrenzt sind, wobei die Förderkolben (12) beweglich im Innenraum (11) der Kartuschen (10) angeordnet sind.

16. Kartuschensystem nach einem der vorangehenden Ansprüche, **dadurch gekennzeichnet, dass**
an der Austragsöffnung (80) ein Austragsrohr (90) umfassend einen statischen Mischer (96) angeordnet ist.

17. Kartuschensystem nach einem der vorangehenden Ansprüche, **dadurch gekennzeichnet, dass**
an dem Gehäuse (70) ein zweites Befestigungsmittel (83), insbesondere ein Innengewinde (83), angeordnet ist, mit dem das Gehäuse (70) an einem an den Kartuschen (10) angeordneten dritten Befestigungsmittel (84), insbesondere einem Außengewinde (84), vorzugsweise lösbar zu befestigen ist.

18. Kartuschensystem nach einem der vorangehenden Ansprüche, **dadurch gekennzeichnet, dass**
die Vorrichtung (40, 140, 240) fluiddicht mit dem Kartuschenkopf (20, 120) abschließt, so dass zumindest zwei Kartuschenöffnungen (30, 130, 230) mit zumindest zwei darüber angeordneten Öffnungen (50, 150, 250) der Vorrichtung (40, 140, 240) zumindest zwei durchgehende, fluiddichte Verbindungen vom Innenraum (11) der Kartuschen (10) in den Innenraum des Gehäuses (70) bilden.

19. Kartuschensystem nach einem der vorangehenden Ansprüche, **dadurch gekennzeichnet, dass**
die Kartuschen (10) durch Drehen oder Verschieben der Vorrichtung (40, 140, 240) verschließbar und/oder öffenbar sind.

20. Vorrichtung (40, 140, 240) für ein Kartuschensystem nach einem der vorangehenden Ansprüche, die drehbar oder verschiebbar auf einem Kartuschenkopf (20, 120) eines Kartuschensystems anordbar ist, **gekennzeichnet durch**
wenigstens zwei Öffnungen (50, 150, 250), die auf dem Kartuschenkopf (20,120) derart verschiebbar sind, dass sie zumindest bereichsweise zwei Kartuschenöffnungen (30, 130, 230) des Kartuschensystems überdecken, wobei an zumindest zwei Öffnungen (50, 150, 250) zumindest jeweils ein Flügel (60, 160, 260) angeordnet ist, der zumindest bereichsweise über die Öffnung (50, 150, 250) ragt.

21. Verfahren zur Synchronisierung von Fluidströmen mit einem Kartuschensystem nach einem der Ansprüche 1 bis 19 oder mit einer Vorrichtung nach Anspruch 20, das dadurch charakterisiert ist, dass
zwei Fluidströme durch die Kartuschenöffnungen (30, 130, 230) und durch die Öffnungen (50, 150, 250) der Vorrichtung (40, 140, 240) gedrückt werden,
die Fluidströme durch die Flügel (60, 160, 260) umgeleitet werden,
bei ungleicher Viskosität der Fluide infolge der unterschiedlichen Strömungsgeschwindigkeit eine unterschiedliche Kraft auf die Flügel (60, 160, 260) ausgeübt wird, dadurch die Vorrichtung (40, 140, 240) gedreht oder verschoben wird, wobei die Öffnungen (50, 150, 250) gegenüber den Kartuschenöffnungen (30, 130, 230) verschoben werden und dadurch die eine Durchtrittsfläche, die durch eine Kartuschenöffnung (30, 130, 230) und die darüber liegende Öffnung (50, 150, 250) begrenzt wird, bei dem niedrig viskoseren Fluid verkleinert wird und die andere Durchtrittsfläche bei dem höher viskosen Fluid vergrößert wird, bis die auf die Flügel (60, 160, 260) einwirkenden Kräfte durch die Veränderung der Fluidströme durch die Durchtrittsflächen gleich groß werden und die Drehbewegung oder die Verschiebung der Vorrichtung (40, 140, 240) gestoppt wird.

22. Verfahren nach Anspruch 21, **dadurch gekennzeichnet, dass**
die Fluidströme, nachdem sie durch die Öffnungen (50, 150, 250) gedrückt werden, in das Gehäuse (70) gedrückt werden und dann durch die Austragsöffnung (80) aus dem Gehäuse (70) heraus gedrückt werden.

23. Verfahren zum Mischen eines Mischguts mit einem Kartuschensystem nach einem der Ansprüche 1 bis 19, **dadurch gekennzeichnet, dass**
das Kartuschensystem zum Mischen von strömenden pastenförmigen Klebstoffen, pastenförmigen Dichtstoffen, pastenförmigen Lebensmitteln, pastenförmigen Dentalmaterialien, pastenförmigen anorganischen Knochenzementen und/oder pastenförmigen Polymethylmethacrylat-Knochenzementen verwendet wird, insbesondere unter Verwendung eines Verfahrens nach einem der Ansprüche 21 bis 22.

24. Verfahren nach Anspruch 23, **dadurch gekennzeichnet, dass**
das gemischte Mischgut appliziert wird.

## Claims

1. A cartridge system for mixing and applying two fluids, in particular of a medical cement, comprising two cartridges (10) and a dispensing opening (80), wherein the cartridges (10) are bordered by cartridge walls and by a cartridge head (20, 120), wherein at least one cartridge opening (30, 130, 230), each for expelling the cartridge contents from both cartridges (10) is provided in the cartridge head (20, 120), **characterized in that**
a device (40, 140, 240) for regulating the fluid flows from the cartridges (10) is arranged on the cartridge head (20, 120), said device comprising at least two wings (60, 160, 260) and at least two openings (50, 150, 250) which overlap, at least partly, with two cartridge openings (30, 130, 230), wherein the device (40, 140, 240) is arranged on the cartridge head (20, 120) in a rotatable or displaceable manner, wherein at least at two openings (50, 150, 250) in each case at least one wing (60, 160, 260) is arranged which projects at least partly over the opening (50, 150, 250) so that a flow through the openings (50, 150, 250) effects, via the wings (60, 160, 260), a force that acts on the device (40, 140, 240) and is parallel to the surface of the openings (50, 150, 250), said force resulting in the device (40, 140, 240) being displaced or rotated with respect to the cartridge head (20, 120), upon which the area of the cartridge openings (30, 130, 230) covered by the device (40, 140, 240) changes, and that a housing (70) is arranged on the cartridge head (20, 120), which housing comprises the dispensing opening (80) and in which the device (40, 140, 240) is arranged in a rotatable or displaceable manner.

2. The cartridge system according to claim 1, **characterized in that** the two cartridges (10) are cylindrical two-component *side-by-side* cartridges (10).

3. The cartridge system according to claim 2, **characterized in that** the device (40, 140, 240) is arranged to be rotatable about an axis parallel the cartridge axis or to be displaceable in a direction perpendicular to the cartridge axis.

4. The cartridge system according to any one of the preceding claims, **characterized in that**
the cartridge head (20, 120) is provided as a planar surface (121).

5. The cartridge system according to claim 4, **characterized in that**
the device (40, 140, 240) is a planar, in particular circular disc (140, 240) that has at least two openings (50, 150, 250) and at least two wings (60, 160, 260) arranged at the openings.

6. The cartridge system according to any one of the preceding claims, **characterized in that**
the cartridge openings (30, 130, 230) connect the interior spaces (11) of the cartridges (10) to the upper side of the cartridge head (20, 120) on which the device (40, 140, 240) is arranged.

7. The cartridge system according to any one of the preceding claims, **characterized in that**
two cartridge openings (30, 130, 230) are provided in the cartridge head (20, 120) and two openings (50, 150, 250) are provided in the device (40, 140, 240), wherein the openings (50, 150, 250) overlap, at least partly, with the cartridge openings (30, 130, 230).

8. The cartridge system according to any one of the preceding claims, **characterized in that**
the wings (60, 160, 260) have an angle of inclination with respect to the openings (50, 150, 250) of 1° to 89°, preferably 30° to 60°, and more preferably 40°to 50°.

9. The cartridge system according to any one of the preceding claims, **characterized in that**
the wings (60, 160, 260) are flat discs, in particular plates.

10. The cartridge system according to any one of the preceding claims, **characterized in that**
the device (40, 240) is mounted on the cartridge head (20) in a displaceable manner, and the wings (60, 260) are inclined in opposite directions with respect to the direction of displacement, wherein the forces acting on the wings (60, 260) as a result of a fluid flow through the openings (50, 250) are directed in opposite directions with respect to the displaceable device (40, 240).

11. The cartridge system according to any one of the claims 1 to 9, **characterized in that** the device (40, 140) is mounted on the cartridge head (20, 120) in a rotatable manner, and the wings (60, 160) are inclined in opposite directions with respect to the rotation axis, wherein the torques acting on the wings (60, 260) as a result of a fluid flow through the openings (50, 250) are directed in opposite directions with respect to the rotation axis of the device (40, 140).

12. The cartridge system according to any one of the preceding claims, **characterized in that**
the housing (70) has an inner diameter that is larger than or equal to the device (40, 140, 240), and the distance of the device (40, 140, 240) from the upper inside of the housing (70) is larger than the perpendicular line from the outside of the wings (60, 160, 260) to the surface of the device (40, 140, 240).

13. The cartridge system according to any one of the preceding claims, **characterized in that**
the housing (70) has on its inside a guidance (71) for the device (40, 140, 240).

14. The cartridge system according to any one of the preceding claims, **characterized in that**
a first fastening means (82), in particular a thread for a dispensing tube (90), is arranged at the dispensing opening (80).

15. The cartridge system according to any one of the preceding claims, **characterized in that**
the cartridges (10) are bordered on the sides opposite from the cartridge head (20, 120) by in each case one feed plunger (12) for expelling the mixture from the cartridges (10), wherein the feed plungers (12) are arranged in a movable manner in the interior space (11) of the cartridges (10) .

16. The cartridge system according to any one of the preceding claims, **characterized in that**
a dispensing tube (90) comprising a static mixer (96) is arranged at the dispensing opening (80).

17. The cartridge system according to any one of the preceding claims, **characterized in that**
a second fastening means (83), in particular an internal thread (83), is arranged on the housing (70), said second fastening means being usable for fastening the housing (70) in a preferably detachable manner to a third fastening means (84), in particular an external thread (84), that is arranged on the cartridges (10).

18. The cartridge system according to any one of the preceding claims, **characterized in that**
the device (40, 140, 240) is flush with the cartridge head (20, 120) in a fluid-tight manner so that at least two cartridge openings (30, 130, 230) together with at least two openings (50, 150, 250) of the device (40, 140, 240) arranged thereabove form at least two continuous fluid-tight connections from the interior space (11) of the cartridges (10) into the interior space of the housing (70).

19. The cartridge system according to any one of the preceding claims, **characterized in that**
the cartridges (10) can be closed and/or opened by rotating or displacing the device (40, 140, 240).

20. A device (40, 140, 240) for a cartridge system according to any one of the preceding claims that can be arranged in a rotatable or displaceable manner on a cartridge head (20, 120) of a cartridge system, **characterized by** at least two openings (50, 150, 250) that can be displaced on the cartridge head (20, 120) in such a manner that they overlap at least partly with two cartridge openings (30, 130, 230) of the cartridge system, wherein at least at two openings (50, 150, 250) in each case at least one wing (60, 160, 260) is arranged that protrudes at least partly over the opening (50, 150, 250).

21. A method for synchronizing fluid flows using a cartridge system according to any one of the claims 1 to 19 or a device according to claim 20, **characterized in that** two fluid flows are pressed through the cartridge openings (30, 130, 230) and through the openings (50, 150, 250) of the device (40, 140, 240),
the fluid flows are redirected by the wings (60, 160, 260), in the case of different viscosities of the fluids due to the different flow velocities, different forces are applied to the wings (60, 160, 260), as a result of which the device (40, 140, 240) is rotated or displaced, whereupon the openings (50, 150, 250) are displaced with respect to the cartridge openings (30, 130, 230) and therefore a passage area that is bordered by a cartridge opening (30, 130, 230) and the opening (50, 150, 250) located thereabove is made smaller for the fluid of low viscosity, and the other passage area is made larger for the fluid of higher viscosity until the forces acting on the wings (60, 160, 260) become equal due to the change in the fluid flows through the passage areas, and the rotary motion or the displacement of the device (40, 140, 240) is stopped.

22. The method according to claim 21, **characterized in that** the fluid flows, once pressed through the openings (50, 150, 250), are pressed into the housing (70) and are then pressed through the dispensing opening (80) out of the housing (70).

23. A method for mixing a mixture using a cartridge system according to any one of the claims 1 to 19, **characterized in that**
the cartridge system is used for mixing flowing pasty adhesives, pasty sealants, pasty foodstuff, pasty dental material, pasty inorganic bone cements and/or pasty polymethylmethacrylate bone cements, in particular by using a method according to any one of the claims 21 to 22.

24. The method according to claim 23, **characterized in that** the mixed mixture is applied.

## Revendications

1. Système de cartouche pour le mélangeage et l'application de deux fluides, en particulier d'un ciment médical, comprenant deux cartouches (10) et un orifice de distribution (80), les cartouches (10) étant délimitées par des parois de cartouche et par une tête de cartouche (20, 120), au moins un orifice de cartouche (30, 130, 230), chacun étant destiné à l'expulsion du contenu de chacune des deux cartouches (10), étant pratiqué dans la tête de cartouche (20, 120), **caractérisé en ce que**
un dispositif (40, 140, 240) de régulation des débits fluidiques depuis les cartouches (10) est disposé sur la tête de cartouche (20, 120), ledit dispositif comprenant au moins deux ailettes (60, 160, 260) et au moins deux orifices (50, 150, 250) au moins en partie superposés avec deux orifices de cartouche (30, 130, 230), le dispositif (40, 140, 240) étant disposé sur la tête de cartouche (20, 120) d'une façon pivotante ou déplaçable, dans chaque cas au moins une ailette (60, 160, 260) au niveau d'au moins deux orifices (50, 150, 250) étant disposée de façon à se projeter au moins partiellement par-dessus l'orifice (50, 150, 250) de sorte qu'un débit à travers les orifices (50, 150, 250) permette d'obtenir, via les ailettes (60, 160, 260), une force agissant sur le dispositif (40, 140, 240) et parallèle à la surface des orifices (50, 150, 250), ladite force entraînant le déplacement ou le pivotement du dispositif (40, 140, 240) par rapport à la tête de cartouche (20, 120), la surface des orifices de cartouche (30, 130, 230) recouverte par le dispositif (40, 140, 240) étant alors modifiée, et **en ce qu'**un boîtier (70) est disposé sur la tête de cartouche (20, 120), ledit boîtier comprenant l'orifice de distribution (80) et ledit dispositif (40, 140, 240) y étant disposé de façon pivotante ou déplaçable.

2. Système de cartouche conforme à la revendication 1, **caractérisé en ce que** les deux cartouches (10) sont des cartouches *côte à côte* bicomposants cylindriques (10).

3. Système de cartouche conforme à la revendication 2, **caractérisé en ce que** le dispositif (40, 140, 240) est disposé de sorte à pouvoir pivoter autour d'un axe parallèle à l'axe de la cartouche ou d'être déplaçable dans une direction perpendiculaire à l'axe de la cartouche.

4. Système de cartouche conforme à l'une quelconque des revendications précédentes, **caractérisé en ce que** :
la tête de cartouche (20, 120) est fournie sous forme d'une surface plane (121).

5. Système de cartouche conforme à la revendication 4, **caractérisé en ce que** le dispositif (40, 140, 240) est un disque plan, en particulier circulaire (140, 240) présentant au moins deux orifices (50, 150, 250) et au moins deux ailettes (60, 160, 260) disposées au niveau des orifices.

6. Système de cartouche conforme à l'une quelconque des revendications précédentes, **caractérisé en ce que** :
les orifices de cartouche (30, 130, 230) relient les espaces intérieurs (11) des cartouches (10) au côté supérieur de la tête de cartouche (20, 120) sur laquelle le dispositif (40, 140, 240) est disposé.

7. Système de cartouche conforme à l'une quelconque des revendications précédentes, **caractérisé en ce que** :
deux orifices de cartouche (30, 130, 230) sont pratiqués dans la tête de cartouche (20, 120) et deux orifices (50, 150, 250) sont pratiqués dans le dispositif (40, 140, 240), les orifices (50, 150, 250) se superposant au moins partiellement avec les orifices de cartouche (30, 130, 230).

8. Système de cartouche conforme à l'une quelconque des revendications précédentes, **caractérisé en ce que** :
les ailettes (60, 160, 260) présentent un angle d'inclinaison par rapport aux orifices (50, 150, 250) compris entre 1° est 89°, préférentiellement entre 30° et 60°, et le plus préférentiellement entre 40° et 50°.

9. Système de cartouche conforme à l'une quelconque des revendications précédentes, **caractérisé en ce que** :
les ailettes (60, 160, 260) sont des disques plats, en particulier des plaques.

10. Système de cartouche conforme à l'une quelconque des revendications précédentes, **caractérisé en ce que** :
le dispositif (40, 240) est installé sur la tête de cartouche (20) d'une façon déplaçable, et
les ailettes (60, 260) sont inclinées dans des directions opposées à la direction de déplacement, les forces agissant sur les ailettes (60, 260) et résultant d'un débit fluidique à travers les orifices (50, 250) étant dirigées dans des directions opposées au dispositif déplaçable (40, 240).

11. Système de cartouche conforme à l'une quelconque des revendications 1 à 9, **caractérisé en ce que** :
le dispositif (40, 140) est installé sur la tête de cartouche (20, 120) d'une façon pivotante, et les ailettes (60, 160) sont inclinées dans des directions opposées à l'axe de rotation, les couples agissant sur les ailettes (60, 260) et résultant d'un débit fluidique à travers les orifices (50, 250) étant dirigés dans des directions opposées à l'axe de rotation du dispositif (40, 140).

12. Système de cartouche conforme à l'une quelconque des revendications précédentes, **caractérisé en ce que** :
le boîtier (70) présente un diamètre interne supérieur ou égal à celui du dispositif (40, 140, 240), et la distance entre le dispositif (40, 140, 240) et la face interne supérieure du boîtier (70) est supérieure à la longueur du segment perpendiculaire entre l'extérieur des ailettes (60, 160, 260) et la surface du dispositif (40, 140, 240).

13. Système de cartouche conforme à l'une quelconque des revendications précédentes, **caractérisé en ce que** :
le boîtier (70) présente sur l'intérieur un guide (71) pour le dispositif (40, 140, 240).

14. Système de cartouche conforme à l'une quelconque des revendications précédentes, **caractérisé en ce que** :
un premier moyen de fixation (82), en particulier un fil pour un tuyau de distribution (90), est disposé au niveau de l'orifice de distribution (80).

15. Système de cartouche conforme à l'une quelconque des revendications précédentes, **caractérisé en ce que** :
les cartouches (10) sont délimitées sur le côté opposé à la tête de cartouche (20, 120) par, dans chaque cas, un piston d'alimentation (12) destiné à expulser le mélange des cartouches (10), les pistons d'alimentation (12) étant disposés de façon déplaçable dans l'espace intérieur (11) des cartouches (10).

16. Système de cartouche conforme à l'une quelconque des revendications précédentes, **caractérisé en ce que** :
un tuyau de distribution (90) comprenant un mélangeur statique (96) est disposé au niveau de l'orifice de distribution (80).

17. Système de cartouche conforme à l'une quelconque des revendications précédentes, **caractérisé en ce que** :
un deuxième moyen de fixation (83), en particulier un fil interne (83), est disposé sur le boîtier (70), ledit deuxième moyen de fixation pouvant être employé pour fixer le boîtier (70) de façon préférentiellement amovible à un troisième moyen de fixation (84), en particulier un fil externe (84), disposé sur les cartouches (10).

18. Système de cartouche conforme à l'une quelconque des revendications précédentes, **caractérisé en ce que** :
le dispositif (40, 140, 240) est en contact intime avec la tête de cartouche (20, 120) de façon imperméable aux fluides de sorte à ce qu'au moins deux orifices de cartouche (30, 130, 230) forment avec au moins deux orifices (50, 150, 250) du dispositif (40, 140, 240) disposés au-dessus de ces derniers au moins deux connexions continues imperméables aux fluides entre l'espace interne (11) des cartouches (10) et l'espace interne du boîtier (70).

19. Système de cartouche conforme à l'une quelconque des revendications précédentes, **caractérisé en ce que** :
les cartouches (10) peuvent être fermées et/ou ouvertes par rotation ou déplacement du dispositif (40, 140, 240).

20. Dispositif (40, 140, 240) pour système de cartouche conforme à l'une quelconque des revendications précédentes qui peut être disposé d'une façon pivotante ou déplaçable sur une tête de cartouche (20, 120) d'un système de cartouche, **caractérisé en ce que** au moins deux orifices (50, 150, 250) peuvent être déplacés sur la tête de cartouche (20, 120) de sorte à se superposer au moins partiellement avec deux orifices de cartouche (30, 130, 230) du système de cartouche, dans chaque cas au moins une ailette (60, 160, 260) au niveau d'au moins deux orifices (50, 150, 250) étant disposée de sorte à dépasser au moins partiellement du dessus de l'orifice (50, 150, 250).

21. Procédé de synchronisation des débits fluidiques employant un système de cartouche conforme à l'une quelconque des revendications 1 à 19 ou dispositif conforme à la revendication 20, **caractérisé en ce que**
deux débits fluidiques sont contraints de traverser les orifices de cartouche (30, 130, 230) et les orifices (50, 150, 250) du dispositif (40, 140, 240),
les débits fluidiques sont redirigés par les ailettes (60, 160, 260),
en cas de viscosité différente des fluides du fait de vitesses de débit différentes, des forces différentes sont appliquées aux ailettes (60, 160, 260), ce qui entraîne le pivotement ou le déplacement du dispositif (40, 140, 240), les orifices (50, 150, 250) étant alors déplacés par rapport aux orifices de cartouche (30, 130, 230) et une zone de passage délimitée par un orifice de cartouche (30, 130, 230) et par l'orifice (50, 150, 250) situé au-dessus de ce dernier étant rétrécie pour le fluide de faible viscosité, tandis que l'autre zone de passage est élargie pour le fluide de viscosité supérieure, jusqu'à ce que les forces agissant sur les ailettes (60, 160, 260) deviennent égales du fait de la modification des débits fluidiques à travers les zones de passage, et le mouvement de rotation ou le déplacement du dispositif (40, 140, 240) s'arrête.

22. Procédé conforme à la revendication 21, **caractérisé en ce que** les débits fluidiques, une fois contraints à traverser les orifices (50, 150, 250), sont contraints à pénétrer dans le boîtier (70) avant d'être contraints à sortir du boîtier (70) à travers l'orifice de distribution (80).

23. Procédé de mélangeage d'un mélange employant un système de cartouche conforme à l'une quelconque des revendications 1 à 19, **caractérisé en ce que** le système de cartouche est employé pour le mélangeage en flux d'adhésifs pâteux, d'agents d'étanchéité pâteux, de denrées alimentaires pâteuses, de matériaux dentaires pâteux, de ciments osseux inorganiques pâteux et/ou de ciments osseux de polyméthacrylate de méthyle pâteux, en particulier en employant un procédé conforme à l'une quelconque des revendications 21 à 22.

24. Procédé conforme à la revendication 23, **caractérisé en ce que** le mélange mélangé est appliqué.
